# EUROPEAN PATENT SPECIFICATION

(11) **EP 4 255 445 B1**
(45) Date of publication and mention of the grant of the patent: **14.05.2025**
(21) Application number: 21816497.8
(22) Date of filing: 02.12.2021
(51) Int. Cl.: A61K 31/706, A61P 31/04, A61K 9/00, A61K 9/08, A61K 47/12

(54) **TILDIPIROSIN FOR THE TREATMENT OF FOOT ROT**
TILDIPIROSIN ZUR BEHANDLUNG VON MODERHINKE
TILDIPIROSINE POUR LE TRAITEMENT DU PIÉTIN

(30) Priority: 03.12.2020 EP 20211553
(43) Date of publication of application: 11.10.2023
(73) Proprietor: Intervet International B.V., 5831 AN Boxmeer (NL)
(72) Inventor: VETTORATO, Luis, Fernando, 04794-000 Sao Paulo (BR); MARQUES DE MORAIS, Maurício, 04583-110 São Paulo (BR); CHIUMMO, Rafael Marin, 55270 Schwabenheim (DE); DE OLIVEIRA ARRIERO AMARAL, Heitor, 04583-110 São Paulo (BR)
(74) Representative: Intervet International B.V.
(86) International application number: PCT/EP2021/083899
(87) International publication number: WO 2022/117701

(56) References cited:
- MERRILL JK ET AL: "Evaluation of the Dosage of Tilmicosin for the Treatment of Acute Bovine Footrot (lnterdigital Phlegmon)", 1 July 1998 (1998-07-01), XP055799736, Retrieved from the Internet <URL:https://doi.org/10.21423/bovine-vol33no1p60-62> [retrieved on 20210429]
- POEHLSGAARD JACOB ET AL: "Visualizing the 16-Membered Ring Macrolides Tildipirosin and Tilmicosin Bound to Their Ribosomal Site", ACS CHEMICAL BIOLOGY, vol. 7, no. 8, 14 May 2012 (2012-05-14), pages 1351 - 1355, XP055799739, ISSN: 1554-8929, DOI: 10.1021/cb300105p
- MAZZEI T ET AL: "Chemistry and mode of action of macrolides.", THE JOURNAL OF ANTIMICROBIAL CHEMOTHERAPY MAR 1993, vol. 31 Suppl C, March 1993 (1993-03-01), pages 1 - 9, XP009527236, ISSN: 0305-7453
- ANGELL JOSEPH W. ET AL: "In vitro susceptibility of contagious ovine digital dermatitis associated Treponema spp. isolates to antimicrobial agents in the UK", VETERINARY DERMATOLOGY., vol. 26, no. 6, 19 October 2015 (2015-10-19), GB, pages 484 - e115, XP055799505, ISSN: 0959-4493, DOI: 10.1111/vde.12269

## Description

### Background

Foot rot disorders in cattle are necrotic diseases that affect the connective tissue of the interdigital region and are caused by bacteria, mainly *Fusobacterium necrophorum.* Foot rot disorders are also commonly found in sheep, goats and swine. These disorders rot away the foot of the animal, specifically, the area between the two toes of the affected animal. They are extremely painful and contagious. Foot infections impact animal productivity and fertility and causes great economic losses, especially those animals raised in intensive feed lot systems.

Tildipirosin is a semi-synthetic macrolide antibiotic and is the active ingredient in Zuprevo^{®}. The IUPAC name for tildipirosin is (4*R*,5*S*,6*S*,7*R*,9*R*,11*E*,13*E*,15*R*,16*R*)-6-[(2*R*,3*R*,4*S*,5*S*,6*R*)-4-(dimethylamino)-3,5-dihydroxy-6-methyloxan-2-yl]oxy-16-ethyl-4-hydroxy-5,9,13-trimethyl-7-(2-piperidin-1-ylethyl)-15-(piperidin-1-ylmethyl)-1-oxacyclohexadeca-11,13-diene-2,10-dione and the structure is

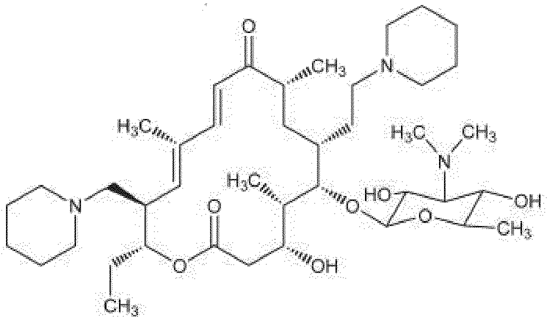

Zuprevo^{®} is a ready-to-use sterile injectable solution containing 18% tildipirosin , a semi-synthetic macrolide antibiotic, for treatment and prophylaxis of Bovine Respiratory Disease. Each mL of Zuprevo^{®} contains 180 mg of tildipirosin as the free base, 82.5 mg citric acid monohydrate and 400 mg propylene glycol, and water qs with citric acid monohydrate added to adjust pH. This pharmaceutical composition is used for the treatment and prevention of bovine respiratory disease (BRD) associated with *Mannheimia haemolytica, Pasteurella multocida* and *Histophilus somni* sensitive to tildipirosin. See Freedom of Information, NADA 141-334, May 14, 2012.

Zuprevo^{®} 4% tildipirosin injectable solution is available for the treatment of swine respiratory disease (SRD) associated with *Actinobacillus pleuropneumoniae, Pasteurella multocida, Bordetella bronchiseptica* and *Haemophilus parasuis.* This solution comprises tildipirosin as a free base in a concentration of 40 mg/mL, propylene glycol, citric acid monohydrate and water. See EPAR Summary for the Public, EMA/166222/2011, updated February 2015.

WO2008/012343 discloses processes to make tildipirosin. WO 2009/013351 discloses solvated and non-solvated crystalline forms of tildipirosin. U.S. Patent No. 6,514,946 discloses the structure of tildipirosin and its use to treat various livestock and poultry diseases.

Recent studies have shown that tildipirosin was rapidly absorbed and slowly eliminated after a single subcutaneous administration in healthy lambs. It was proposed that tildipirosin could be used for the treatment and prevention of respiratory bacterial infections in sheep (see Abu-Basha, EA, et al., Pharmacokinetics and bioavailability of tildipirosin following intravenous and subcutaneous administration in sheep. J vet Pharmacol Therap. 2020; 00: 1- 7. https://doi.org/10.1111/jvp.12901).

Merrill Jk ET AL: "Evaluation of the Dosage of Tilmicosin for the Treatment of Acute Bovine Footrot (Interdigital Phlegmon)", 1 July 1998, discloses a 16-membered macrolide tilmicosin for the treatment of acute bovine foot rot.

None of these references discloses the use of tildipirosin to treat or prevent foot rot diseases.

### Summary of the Invention

An embodiment of the invention is an injectable pharmaceutical composition comprising tildipirosin, a salt or solvate thereof and a pharmaceutically acceptable carrier for use in the treatment and/or prevention of foot rot disease in a bovid animal.

### Description of the Figures

Figure 1 presents the lameness data from the tildipirosin group and the control group.
Figure 2 presents the lesion data from the tildipirosin group and the control group.

### Detailed Description

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy.

The subject invention is the use of an injectable pharmaceutical composition comprising tildipirosin for the treatment and prevention of foot rot. The use of a single subcutaneous dose of tildipirosin injectable solution at the dose of 4 mg of tildipirosin per kg of bodyweight (BW) has been demonstrated to effectively treat naturally occurring foot rot.

The treatment of foot rot is useful on intensively reared beef cattle such as those in feedlot confinement operations. The treatment is intended to reduce hoof lesions and lameness, reduce infections and prevent new infections from bacteria such as F. *necrophorum.* The invention can have significant impact on farm productivity and animal welfare. The invention is convenient to the farm management since it consists of a single treatment which reduces the need of restraining animals. Furthermore, tildipirosin is also used to treat and prevent Bovine Respiratory Disease (BRD), so that a single treatment can help control two of the most important diseases on intensively reared beef cattle.

An embodiment of the invention is an injectable pharmaceutical composition comprising tildipirosin, a salt or solvate thereof and a pharmaceutically acceptable carrier for use in the treatment and/or prevention of foot rot disease in a bovid animal.

In an embodiment of the invention, the pharmaceutically acceptable carrier comprises one or more excipients.

In an embodiment of the invention, the injectable pharmaceutical composition is a solution or a suspension.

In an embodiment of the invention, the injectable pharmaceutical composition is a solution.

In an embodiment of the invention, the excipients are one or more solvents.

In an embodiment of the invention, the solvents are propylene glycol, water or mixtures thereof.

In an embodiment of the invention, the tildipirosin is the form of the free base.

In an embodiment of the invention, the pharmaceutical composition further comprises an acid, preferably citric acid.

In an embodiment of the invention, the dose of tildipirosin is from 1 mg/kg to 10 mg/kg of body weight of the animal or from 3 mg/kg to7 mg/kg or preferably 4 mg/kg.

In an embodiment of the invention, the animal is a bovid, such as a sheep or a goat.

In an alternative embodiment of the invention, the animal is a sheep.

In an alternative embodiment of the invention, the animal is a goat.

In an embodiment of the invention, the concentration of tildipirosin is from 10% w/v to 25% *w*/*v* or from 15% *w*/*v* to 20 % *w*/*v* or preferably 18% w/v.

In an embodiment of the invention, the concentration of tildipirosin is from 0.1% *w*/*v* to 9.9% *w*/*v* or from 2% w/v to 6 % w/v or preferably 4% w/v.

An embodiment of the invention is an injectable pharmaceutical composition for use in the treatment and/or prevention of foot rot disease in a bovid animal, wherein each mL of the composition comprises
a) 180 mg of tildipirosin as the free base;
b) 82.5 mg citric acid monohydrate;
c) 400 mg propylene glycol; and
d) water qs.

In an embodiment of the invention, the animal is a bovid.

In an embodiment of the invention, the injectable pharmaceutical composition is a suspension.

In an embodiment of the invention, the injectable pharmaceutical composition comprises a salt or a solvate of tildipirosin.

An alternative embodiment of the invention is a method of treating foot rot in a bovid animal comprising administering a pharmaceutical composition comprising an effective amount of tildipirosin to the animal.

In another embodiment, the pharmaceutical composition is administered intravenously. In another embodiment, the method comprises a single administration of the pharmaceutical composition.

In another embodiment, the effective amount of tildipirosin is from 1 mg/kg to 10 mg/kg of body weight of the animal.

In another embodiment, wherein the effective amount of tildipirosin is from 3 mg/kg to 7 mg/kg, preferably 4 mg/kg.

In another embodiment, the animal is a bovid, such as a sheep or a goat.

In another embodiment, the pharmaceutical composition further comprises one or more excipients.

In another embodiment, the tildipirosin is in the form of the freebase.

In another embodiment, the pharmaceutical composition comprises a solvent selected from propylene glycol, water or mixtures thereof.

In another embodiment, the pharmaceutical composition comprises an acid.

In another embodiment, the acid is citric acid.

In another embodiment, the use is a single injection.

### EXAMPLE 1

This study demonstrated the efficacy of tildipirosin injectable solution (Zuprevo^{®}) for use in bovine animals, given subcutaneously at one single dose of 4 mg of the active ingredient/kg body weight (BW) against naturally occurring foot rot.

Thirty animals were enrolled to the Control Group and another thirty animals were enrolled in the Tildipirosin treated Group. The tildipirosin-treated group received a single dose, subcutaneously, while the control group was treated with a saline sterile solution. Both groups (treated and control) were housed in the same paddocks under typical Brazilian feedlot conditions.

Sixty animals were included in the trial in two different phases, according to the number of available animals that fulfill the inclusion criteria. The study was masked. That is the study personnel making post-treatment clinical assessments including lameness did not know the treatment assignments. The animals were randomized and evenly distributed across the treatment groups according to 15 blocks of 2 animals each in both inclusion dates (there were 2 inclusion dates, for a total of 60 animals).

The effectiveness of tildipirosin for the treatment of foot rot was evaluated by comparing the proportion of treatment success/improvement in the tildipirosin-treated group to the saline-treated control group on Day 7.

There were no abnormal health observations before or after treatment. One adverse event was observed with one animal of the tildipirosin-treated group that had a broken toenail on Day 7. According to the investigator, the adverse event was not related to the tildipirosin treatment, and this animal was removed from the study.

The lameness and the lesion scoring are described in Tables 1 and 2 below.

**Table 1 Lameness Scoring guide**

| Score value | Clinical Description | Assessment Criteria |
|---|---|---|
| 1 | Normal | Animal stands and walks with a level-back posture and clinically normal gait. |
| 2 | Mildly lame | Animal stands with a level-back posture but develops an arched-back posture during walking. The gait remains clinically normal. |
| 3 | Moderately lame | Animal has an arched-back posture that is evident during standing and walking. Animal has a short-strided gait in 1 or more limbs. |
| 4 | lame | Animal always has an arched-back posture and gait is one deliberate step at a time. Animal favors one or more limbs or feet. |
| 5 | Severely lame | Animal additionally demonstrates an inability or extreme reluctance to bear weight on one or more limbs or feet. |

**Table 2 Lesion Scoring guide**

| Score value | Lesion Description |
|---|---|
| 1 | No abnormality observed. |
| 2 | Slight to moderate swelling present. Evidence of healing lesion with granulation tissue present throughout the majority of the lesion. |
| 3 | Severe swelling with no necrotic lesion; or slight swelling with small interdigital necrotic lesion covering up to 10% of the interdigital space. |
| 4 | Small to medium size necrotic lesion covering >10% up to 50% of the interdigital space with moderate to severe swelling. |
| 5 | Large to very large interdigital necrotic lesion covering >50% up to100% of the interdigital space with moderate to severe swelling. |

The animals' lesion and lameness score were assessed by the investigator on Days -1 and 0 of the study. To qualify for the effectiveness portion of the study, animals were diagnosed with foot rot by meeting the following criteria:
- Lameness Score ≥ 3 in at least one foot for 2 consecutive days.
- Lesion Score ≥ 2 in at least one foot for 2 consecutive days.
- Clinically healthy (excepting lameness)
- Body weight (348-499 kg body weight)
- Age (20-24 months)

On Day 0, before treatment, bacteriological samples were collected from the infected hooves of the enrolled cattle. Lesions and lameness were also assessed on Days 2, 4 and 7. Treatment success, improvement, or failure was determined on Day 7 and was based on lameness and lesion score compared to Day 0. On Day 7, another bacteriological sample was collected from all animals.

Bacteriological samples were collected on a sterile scraper and on a sterile swab from the border of healthy and necrotic tissue (around the hoof lesion) of the infected hooves of the enrolled cattle. The tip of the scraper, containing discharge from the lesions, was fractured off into 15 ml tubes containing 7 ml of a semi solid transport media formulated exclusively to anaerobic bacteria and the swab was discharged in a semi solid transport. The tubes were kept under refrigeration (4 to 8 °C) until they arrive into the laboratory. At the laboratory, the samples grew in culture media appropriated to F. *necrophorum* and *Dichelobacter nodosus* isolation. Isolates were identified using confirmatory biochemical tests and polymerase chain reaction (PCR) specific to F. *necrophorum* and *D. nodosus* species.

The tildipirosin-treated group demonstrated decreased symptoms scores for both lameness and lesion. In the treated group, the animals had an average lameness score of 3.1 on Day 0. The lameness score of this group decreased to 1.3 on Day 7. For the lesion score, the treated group animals had an average score of 2.3 on Day 0 which decreased to a score of 1.9 on Day 7. In contrast, the control group's average lameness score of 3.1 on Day 0 decreased to only 2.4 on Day 7. Their average lesion score of 2.3 on Day 0 increased slightly to a score of 2.4 on Day 7.

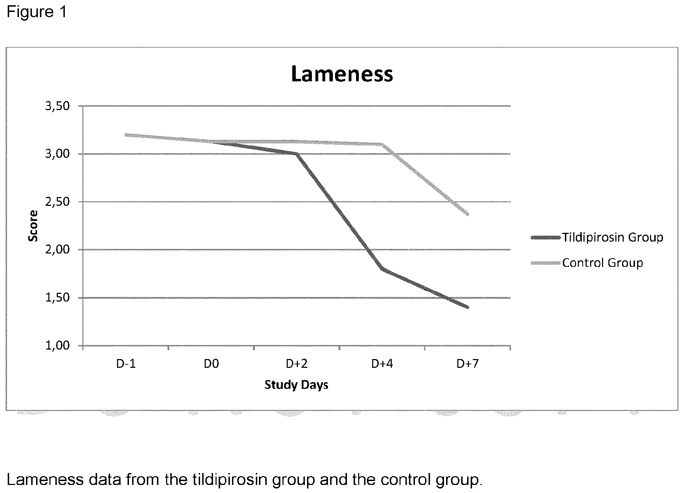

Figure 1 presents the lameness score data. Both the treated group and the control group had the same mean score (3.1). After treatment each group behaved differently. The score of the tildipirosin treated animals decreased to 1.3, while the score of the control group stayed in 2.4. Clearly, the tildipirosin treatment produced a better score evaluation in the tildipirosin treated animals as shown in Figure 1.

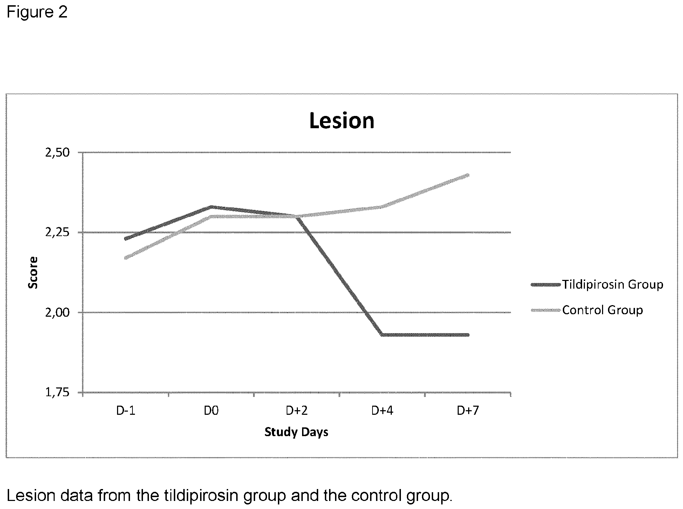

Figure 2 displays the lesion score data. Before treatment, the lesion score was similar for both groups: tildipirosin-treated group (2.3) and control group (2.2). However, there is a visible difference in the behavior of each group following evaluations at Day 7. The control group increased the lesion score (2.4), in other words it got worse, and the tildipirosin treated animals decreased the lesion score (1.9), getting closer to a clinical resolution.

The bacterial evaluation was not conclusive, although more animals treated with tildipirosin that were positive to PCR identification of *Fusobacterium necrophorum* before treatment administration became negative after treatment and also less tildipirosin treated animals that were previously negative became positive, both in comparison to the control group.

## Claims

1. An injectable pharmaceutical composition comprising tildipirosin, a salt or solvate thereof and a pharmaceutically acceptable carrier for use in a treatment and/or prevention of foot rot disease in a bovid animal.

2. The injectable pharmaceutical composition for use according to claim 1, wherein the pharmaceutically acceptable carrier comprises one or more excipients.

3. The injectable pharmaceutical composition for use according to anyone of claims 1-2, wherein the injectable pharmaceutical composition is a solution or a suspension.

4. The injectable pharmaceutical composition for use according to claim 3, wherein the injectable pharmaceutical composition is a solution.

5. The injectable pharmaceutical composition for use according to claim 4, wherein the excipients are one or more solvents.

6. The injectable pharmaceutical composition for use according to claim 5, wherein the solvents are propylene glycol, water or mixtures thereof.

7. The injectable pharmaceutical composition for use according to anyone of claims 1-6, wherein the tildipirosin is the form of the free base.

8. The injectable pharmaceutical composition for use according to claim 7, wherein the pharmaceutical composition further comprises an acid.

9. The injectable pharmaceutical composition for use according to claim 8, wherein the acid is citric acid.

10. The injectable pharmaceutical composition for use according to anyone of claims 1-9, wherein the dose of the tildipirosin is from 1 mg/kg to 10 mg/kg of body weight of the animal or from 3 mg/kg to 7 mg/kg or preferably 4 mg/kg.

11. The injectable pharmaceutical composition for use according to claim 4, wherein the concentration of the tildipirosin is from 10% w/v to 25% w/v, from 15% w/v to 20 % w/v, preferably 18% w/v.

12. An injectable pharmaceutical composition for use in a treatment and/or prevention of foot rot disease in a bovid animal, wherein each mL of the composition comprises
a) 180 mg of tildipirosin as the free base;
b) 82.5 mg citric acid monohydrate;
c) 400 mg propylene glycol; and
d) water qs.

13. The injectable pharmaceutical composition for use according to claim 3, wherein the injectable pharmaceutical composition is a suspension.

14. The injectable pharmaceutical composition for use according to claim 13, comprising a salt or a solvate of tildipirosin.

15. The injectable pharmaceutical composition for use according to anyone of claims 1-14, wherein the use is a single injection.

## Patentansprüche

1. Eine injizierbare pharmazeutische Zusammensetzung, die Tildipirosin, ein Salz oder Solvat davon und einen pharmakologisch akzeptablen Träger umfasst, zur Anwendung in der Behandlung und/oder Vorbeugung von Moderhinke bei Rindern.

2. Die injizierbare pharmazeutische Zusammensetzung zur Anwendung gemäß Anspruch 1, wobei der pharmakologisch akzeptable Träger ein oder mehrere Hilfsstoffe umfasst.

3. Die injizierbare pharmazeutische Zusammensetzung zur Anwendung gemäß einem der Ansprüche 1-2, wobei die injizierbare pharmazeutische Zusammensetzung eine Lösung oder eine Suspension ist.

4. Die injizierbare pharmazeutische Zusammensetzung zur Anwendung gemäß Anspruch 3, wobei die injizierbare pharmazeutische Zusammensetzung eine Lösung ist.

5. Die injizierbare pharmazeutische Zusammensetzung zur Anwendung gemäß Anspruch 4, wobei die Hilfsstoffe ein oder mehrere Lösungsmittel sind.

6. Die injizierbare pharmazeutische Zusammensetzung zur Anwendung gemäß Anspruch 5, wobei die Lösungsmittel Propylenglykol, Wasser oder Mischungen davon sind.

7. Die injizierbare pharmazeutische Zusammensetzung zur Anwendung gemäß einem der Ansprüche 1-6, wobei das Tildipirosin in Form der freien Base vorliegt.

8. Die injizierbare pharmazeutische Zusammensetzung zur Anwendung gemäß Anspruch 7, wobei die pharmazeutische Zusammensetzung zusätzlich eine Säure umfasst.

9. Die injizierbare pharmazeutische Zusammensetzung zur Anwendung gemäß Anspruch 8, wobei die Säure Zitronensäure ist.

10. Die injizierbare pharmazeutische Zusammensetzung zur Anwendung gemäß einem der Ansprüche 1-9, wobei die Dosis des Tildipirosins von 1 mg/kg bis 10 mg/kg Körpergewicht des Tieres oder von 3 mg/kg bis 7 mg/kg oder vorzugsweise 4 mg/kg beträgt.

11. Die injizierbare pharmazeutische Zusammensetzung zur Anwendung gemäß Anspruch 4, wobei die Konzentration des Tildipirosins von 10% w/v bis 25% w/v, von 15% w/v bis 20% w/v, vorzugsweise 18% w/v beträgt.

12. Eine injizierbare pharmazeutische Zusammensetzung zur Anwendung in der Behandlung und/oder Vorbeugung von Moderhinke bei Rindern, wobei jedes mL der Zusammensetzung enthält:
a) 180 mg Tildipirosin als freie Base;
b) 82,5 mg Zitronensäure-Monohydrat;
c) 400 mg Propylenglykol; und
d) Wasser qs.

13. Die injizierbare pharmazeutische Zusammensetzung zur Anwendung gemäß Anspruch 3, wobei die injizierbare pharmazeutische Zusammensetzung eine Suspension ist.

14. Die injizierbare pharmazeutische Zusammensetzung zur Anwendung gemäß Anspruch 13, die ein Salz oder ein Solvat von Tildipirosin umfasst.

15. Die injizierbare pharmazeutische Zusammensetzung zur Anwendung gemäß einem der Ansprüche 1-14, wobei die Anwendung eine Einzelinjektion ist.

## Revendications

1. Composition pharmaceutique injectable comprenant de la tildipirosine, un sel ou un solvate correspondant et un support pharmaceutiquement acceptable pour une utilisation dans un traitement et/ou la prévention du piétin chez un bovidé.

2. Composition pharmaceutique injectable pour une utilisation selon la revendication 1, le support pharmaceutiquement acceptable comprenant un ou plusieurs excipients.

3. Composition pharmaceutique injectable pour une utilisation selon l'une quelconque des revendications 1-2, la composition pharmaceutique injectable étant une solution ou une suspension.

4. Composition pharmaceutique injectable pour une utilisation selon la revendication 3, la composition pharmaceutique injectable étant une solution.

5. Composition pharmaceutique injectable pour une utilisation selon la revendication 4, les excipients étant un ou plusieurs solvants.

6. Composition pharmaceutique injectable pour une utilisation selon la revendication 5, les solvants étant le propylèneglycol, l'eau ou des mélanges correspondants.

7. Composition pharmaceutique injectable pour une utilisation selon l'une quelconque des revendications 1-6, la tildipirosine étant la forme de la base libre.

8. Composition pharmaceutique injectable pour une utilisation selon la revendication 7, la composition pharmaceutique comprenant en outre un acide.

9. Composition pharmaceutique injectable pour une utilisation selon la revendication 8, l'acide étant l'acide citrique.

10. Composition pharmaceutique injectable pour une utilisation selon l'une quelconque des revendications 1-9, la dose de la tildipirosine étant de 1 mg/kg à 10 mg/kg de poids corporel de l'animal ou de 3 mg/kg à 7 mg/kg ou préférablement 4 mg/kg.

11. Composition pharmaceutique injectable pour une utilisation selon la revendication 4, la concentration de la tildipirosine étant de 10 % p/v à 25 % p/v, de 15 % p/v à 20 % p/v, préférablement 18 % p/v.

12. Composition pharmaceutique injectable pour une utilisation dans un traitement et/ou la prévention du piétin chez un bovidé, chaque mL de la composition comprenant
a) 180 mg de tildipirosine en tant que base libre ;
b) 82,5 mg de monohydrate d'acide citrique ;
c) 400 mg de propylèneglycol ; et
d) de l'eau qs.

13. Composition pharmaceutique injectable pour une utilisation selon la revendication 3, la composition pharmaceutique injectable étant une suspension.

14. Composition pharmaceutique injectable pour une utilisation selon la revendication 13, comprenant un sel ou un solvate de tildipirosine.

15. Composition pharmaceutique injectable pour une utilisation selon l'une quelconque des revendications 1-14, l'utilisation étant une injection unique.
